**Europäisches Patentamt**

**European Patent Office**

**Office européen des brevets**

(19)

(11) Veröffentlichungsnummer: **0 124 744 B1**

(12) # EUROPÄISCHE PATENTSCHRIFT

(45) Veröffentlichungstag der Patentschrift:
**03.06.87**

(21) Anmeldenummer: **84103306.1**

(22) Anmeldetag: **26.03.84**

(51) Int. Cl.⁴: **C 07 C 5/08,** C 07 C 7/163,
B 01 J 23/89, B 01 J 23/88,
B 01 J 23/85, B 01 J 23/72

(54) **Hydrierkatalysator, Verfahren zu seiner Herstellung und seine Verwendung.**

(30) Priorität: **05.04.83 DE 3312252**

(43) Veröffentlichungstag der Anmeldung:
**14.11.84 Patentblatt 84/46**

(45) Bekanntmachung des Hinweises auf die Patenterteilung:
**03.06.87 Patentblatt 87/23**

(84) Benannte Vertragsstaaten:
**AT DE GB NL**

(56) Entgegenhaltungen:
**DE - A - 2 849 026
FR - A - 1 505 663
US - A - 3 200 167
US - A - 3 325 556
US - A - 3 651 167
US - A - 4 101 451**

(73) Patentinhaber: **EC ERDÖLCHEMIE GMBH,
Postfach 75 20 02, D-5000 Köln 71 (DE)**

(72) Erfinder: **Müller, Hans Joachim, Dr., Heidberg 10,
D-5090 Leverkusen (DE)**
Erfinder: **Reinhardt, Horst, Dipl.-Ing., Palmenweg 60,
D-5010 Bergheim 3 (DE)**
Erfinder: **Mentzen, Heinz, Herriger Weg 45,
D-5024 Pulheim 4 (DE)**

(74) Vertreter: **Schumacher, Günter, Dr. et al, c/o Bayer AG
Konzernverwaltung RP Patentabteilung,
D-5090 Leverkusen 1 Bayerwerk (DE)**

## Beschreibung

Die vorliegende Erfindung betrifft Hydrierkatalysatoren, die besonders durch einen Gehalt an Kaliumoxid ausgezeichnet sind, ein Verfahren zu ihrer Herstellung und die Verwendung dieser Hydrierkatalysatoren zur selektiven Hydrierung von acetylenischen zu olefinischen Verbindungen.

Bei der thermischen oder katalytischen Spaltung von Naphtha, Leichtbenzin, Erdgas oder anderen kohlenwasserstoffhaltigen Einsatzstoffen entstehen im allgemeinen neben gesättigten Verbindungen eine Vielzahl von ungesättigten Verbindungen. Solche ungesättigten Verbindungen können eine oder zwei olefinische Doppelbindungen und/oder acetylenisch ungesättigte Bindungen enthalten. Die von den Spaltanlagen (Crackern) abfliessenden Produktgemische werden in ein oder mehreren Trennoperationen in verschiedene Fraktionen und zugleich oder anschliessend in einzelne gewünschte Wertstoffe aufgetrennt. Die Fraktionen werden dabei im allgemeinen so aufgefangen, dass sie überwiegend Kohlenwasserstoffe mit der gleichen Anzahl von C-Atomen enthalten.

So fallen beispielsweise beim Einsatz eines Steam-Crackers zur Herstellung von Ethylen $C_4$-Kohlenwasserstoffströme an, die geradkettige und verzweigte Alkane, Alkene, Alkadiene und Alkine enthalten. Zur Gewinnung von Butadien-1,3 als einem Beispiel für einen Wertstoff und den Crackprodukten wurden verschiedene Verfahren entwickelt. So erfolgt beispielsweise die Gewinnung des Butadien-1,3 aus solchen $C_4$-Kohlenwasserstoffströmen durch Extraktions- bzw. Extraktivdestillationsverfahren. Hierbei werden acetylenische Beimengungen entweder vor der eigentlichen Butadien-1,3-Gewinnung durch eine selektive Hydrierung in überwiegend olefinische bzw. diolefinische Kohlenwasserstoffe übergeführt oder aber extraktiv-destillativ abgetrennt. Hierbei kann sowohl der Gesamt-$C_4$-Strom vor dem Einsatz in die eigentliche Butadien-1,3-Gewinnung als auch der bei der Butadiengewinnung abgetrennte $C_4$-Strom, der die acetylenischen Verbindungen enthält, selektiv hydriert werden.

Für die Selektivhydrierung wurden in der Vergangenheit verschiedene Verfahren entwickelt. Hierbei wurden ein- oder mehrstufige Reaktoren eingesetzt, und der zu hydrierende Strom wurde in der Misch-, Riesel-, Flüssig- oder Gasphase behandelt. Als Katalysatoren wurden hierzu Edelmetalle der VIII. Nebengruppe des Periodensystems der Elemente, vielfach Palladium, sowie andere katalytisch wirksame Metalle, beispielsweise Kupfer, eingesetzt. Typische Verfahren für diese Selektivhydrierung sind beispielsweise beschrieben in US 3 898 298, US 3 912 789 und DE-OS 29 13 209. Trotz vielfältiger Versuche, die Selektivität der bisherigen Verfahren zu steigern, werden in dieser Hinsicht noch nicht alle Erwartungen erfüllt.

Hydrierkatalysatoren mit Cu als der hydrieraktiven Komponente werden beispielsweise in FR 1 505 663 beschrieben. Die Katalysatoren werden mit einem beliebigen Alkalimetallhydroxid imprägniert, calciniert und danach mit Wasser ausgelaugt. Auch in US 4 101 451 werden Kupferkatalysatoren beschrieben, die eine Dotierung von NaOH erhalten. Andere Alkalimetallhydroxide werden ohne weitere Differenzierung ebenfalls für einsetzbar gehalten.

Die Problematik der bisherigen Hydrierverfahren liegt beispielsweise darin, dass ein möglichst hoher Umsatz der acetylenischen Verbindungen, beispielsweise des Vinylacetylens, selektiv zu Olefinen, beispielsweise zum Butadien-1,3, erreicht werden soll, wobei eine Weiterhydrierung des Olefins (beispielsweise des Butadiens-1,3) weitestgehend unterbleiben soll. Bei der Selektivhydrierung von Roh-$C_4$-Strömen nach bisherigen Verfahren werden Umsätze der acetylenischen Verbindungen bis über 90% erzielt, jedoch muss dabei in der Regel ein Butadien-1,3-Verlust von 2–5% hingenommen werden.

Es wurden nun neue Hydrierkatalysatoren aus 0,1 – 60 Gew.-% eines hydrieraktiven Metalles oder einer hydrieraktiven Metallverbindung der VIII. Nebengruppe des Periodensystens der Elemente auf einem inerten Träger mit 0,1 – 10 Gew.-% $K_2O$ sowie gegebenenfalls 0,001 –10 Gew.-% eines Zusatzstoffes aus der Gruppe Calcium, Magnesium, Barium, Lithium, Natrium, Vanadium, Silber, Gold, Kupfer oder Zink, alles bezogen auf das Gesamtgewicht des Katalysators, gefunden, wobei die $K_2O$-Dotierung auf eine Katalysator-Vorstufe aus der hydrieraktiven Komponente, dem Träger und gegebenenfalls dem Zusatzstoff aufgebracht wird.

Als hydrieraktive Metalle, die auch einer hydrieraktiven Metallverbindung zugrundeliegen können, seien die Metalle der VIII. Nebengruppe des Periodensystems der Elemente genannt, wie Eisen, Kobalt, Nickel, Ruthenium, Rhodium, Palladium, Osmium, Iridium, Platin. Gegebenenfalls können auch zwei oder mehr der genannten Metalle in den erfindungsgemässen Hydrierkatalysatoren eingesetzt sein. Für den Fall, dass es sich um hydrieraktive Metallverbindungen handelt, seien beispielsweise sulfidische oder oxidische Verbindungen genannt. Es ist jedoch bevorzugt, die hydrieraktiven Metalle in elementarer Form einzusetzen. Unter den Metallen sind wiederum die Edelmetalle der VIII. Nebengruppe des Periodensystems bevorzugt. In besonders bevorzugter Weise sei Palladium erwähnt.

Im allgemeinen enthalten die erfindungsgemässen Hydrierkatalysatoren 0,1 – 60 Gew.-% des hydrieraktiven Metalls oder der hydrieraktiven Metallverbindung, bezogen auf das Gesamtgewicht des Katalysators. Für den Fall der Verwendung von hydrieraktiven Metallen in elementarer Form beträgt deren bevorzugte Konzentration 0,1 – 20 Gew.-% des Gesamtgewichts des Katalysators.

Für den Fall des Einsatzes von Edelmetallen wird eine Konzentration im unteren Teil des genannten Bereiches gewählt, beispielsweise 0,1 –

5 Gew.-%, bevorzugt 0,1 – 1 Gew.-% und besonders bevorzugt 0,2 – 0,5 Gew.-%. Als Trägermaterial seien beispielsweise genannt: $Al_2O_3$, $SiO_2$, Alumosilikate, Kieselgur, Bariumsulfat oder Aktivkohle. Diese Trägermaterialien können in Form von Kugeln, Extrudaten, Tabletten, Hohlkörpern eingesetzt werden.

Neben der hydrieraktiven Komponente und dem Trägermaterial können die Hydrierkatalysatoren noch Zusatzstoffe enthalten, wie Calcium, Magnesium, Barium, Lithium, Natrium, Vanadinium, Silber, Gold, Kupfer oder Zink, gegebenenfalls in Form ihrer Oxide oder in Form anderer Verbindungen. Diese Zusatzstoffe können in Mengen bis zu 10 Gew.-% des Gesamtkatalysatorgewichts, beispielsweise in einer Menge von 0,001 – 10 Gew.-% vorhanden sein.

Die erfindungsgemässen Katalysatoren weisen eine Dotierung mit $K_2O$ in einer Menge von 0,1 – 10 Gew.-%, bevorzugt 0,3 – 3 Gew.-%, besonders bevorzugt 0,5 – 2 Gew.-% des gesamten Katalysatorgewichts auf. Die $K_2O$-Dotierung wird auf eine Katalysator-Vorstufe aufgebracht, die aus der hydrieraktiven Komponente, dem Träger und gegebenenfalls dem Zusatzstoff besteht.

Die Erfindung betrifft weiterhin ein Verfahren zur Herstellung der neuen Hydrierkatalysatoren, das dadurch gekennzeichnet ist, dass man 0,1 – 60 Gew.-% eines hydrieraktiven Metalls oder einer hydrieraktiven Metallverbindung und gegebenenfalls 0,001 – 10 Gew.-% eines Zusatzstoffes in an sich bekannter Weise auf einen inerten Träger bringt und anschliessend 0,1 – 10 Gew.-% einer wasserlöslichen Kaliumverbindung, gerechnet als $K_2O$, auf die beschriebene Katalysator-Vorstufe bringt, trocknet und durch Kalzinierung fixiert, wobei alle Gewichtsangaben auf das Gesamtgewicht der Hydrierkatalysatoren bezogen sind.

Als Kaliumverbindung kann jede eingesetzt werden, die unter den Bedingungen der nachfolgenden Kalzinierung Kaliumoxid $K_2O$ ergibt, beispielsweise Kaliumhydroxid, Kaliumcarbonat, Kaliumhydrogencarbonat, Kaliumnitrat, Salze organischer Säuren, wie Kaliumacetat, Kaliumpropionat oder Kaliumbenzoat sowie Kaliumphenolat. In bevorzugter Weise werden Kaliumhydroxid oder Kaliumnitrat eingesetzt. Das Aufbringen einer solchen Kaliumverbindung erfolgt zweckmässigerweise aus wässriger Lösung, beispielsweise durch Aufsprühen auf die Katalysator-Vorstufe oder durch Tränken derselben. In bevorzugter Weise wird die Katalysator-Vorstufe durch Tränken mit der Kaliumverbindung versetzt.

Zur Aufbringung der Kaliumverbindung wird je nach gewünschter $K_2O$-Dotierung eine 0,1 – 10 Gew.-%, bevorzugt 0,2 – 5 gew.-%ige, besonders 0,3 – 2 gew.-%ige Lösung des Kaliumsalzes eingesetzt. Für den Fall des Aufbringens der Kaliumverbindung durch Tränken der Katalysator-Vorstufe kann beispielsweise eine Menge von 0,5 – 20 Vol.- Teilen Lösung der Kaliumverbindung, bevorzugt 1 – 8 Teilen Lösung pro Vol.- Teil Katalysator-Vorstufe ausgegangen werden. Dieses Gemisch aus Katalysator-Vorstufe und der Lösung der Kaliumverbindung kann sodann beispielsweise in einem Rotationskolben-Verdampfer bei einer Temperatur von 340 – 400 K und leichtem Unterdruck von 50 – 200 mbar weiterbehandelt, wobei der Wasseranteil der Lösung langsam verdampft wird. Nachfolgend kann im gleichen Rotationskolben-Verdampfer bei etwas erhöhter Temperatur, beispielsweise 373 – 423 K, bevorzugt bei 383 – 403 K und ebenfalls vermindertem Druck, beispielsweise 5 – 100 mbar, bevorzugt 10 – 80 mbar, getrocknet werden. Nach der Trocknung wird der imprägnierte Katalysator für etwa 3 – 8 Stunden, bevorzugt 4 – 6 Stunden bei einer Temperatur von 673 – 1273 K, bevorzugt 673 – 973 K und besonders bevorzugt bei 723 – 823 K kalziniert.

Die Menge des Kaliumsalzes in der zum Sprühen oder zum Tränken benutzten wässrigen Lösung wird so bemessen, dass das bei der Kalzinierung entstehende Kaliumoxid $K_2O$ im oben genannten Bereich liegt.

Die $K_2O$-Dotierung ist grundsätzlich wirksam für alle Hydrierkatalysatoren, unabhängig von ihrem Porenvolumen und ihrer inneren Oberfläche. Für die weiter unten beschriebene selektive Hydrierung von acetylenischen zu olefinischen Verbindungen hat sich ein $K_2O$-dotierter Hydrierkatalysator mit folgenden Eigenschaften besonders bewährt:

als hydrieraktive Metalle werden eingesetzt: Pd
als Träger werden eingesetzt: $Al_2O_3$
das Porenvolumen beträgt: 0,2–0,3 $cm^3/g$
die Oberfläche beträgt: 2 –5 $m^2/g$

Ein in der beschriebenen Weise hergestellter Hydrierkatalysator wird sodann mit einem Inertgas sauerstofffrei gespült. Als Inertgas können beispielsweise Stickstoff oder Methan verwendet werden. Danach kann dieser Katalysator mit Wasserstoff bei 323 – 523 K aktiviert werden. Als Wasserstoff können hierbei reiner Wasserstoff, Wasserstoff-Methan-Gemische, Wasserstoff-Stickstoff-Gemische oder sonstige Wasserstoff-Inertgas-Gemische eingesetzt werden.

Die erfindungsgemässen Katalysatoren eignen sich in vorteilhafter Weise zur selektiven Hydrierung von acetylenischen Verbindungen zu olefinischen Verbindungen. Hierbei werden Dreifachbindungen mit hoher Selektivität in Doppelbindungen übergeführt, ohne dass die entstandenen oder ursprünglich vorhandenen olefinischen Doppelbindungen weiter hydriert werden. Solche acetylenischen Verbindungen haben beispielsweise 2 – 6, bevorzugt 3 – 5 und besonders bevorzugt 4 C-Atome und gegebenenfalls zusätzlich eine olefinische Doppelbindung, wie Acetylen, Methylacetylen, Ethylacetylen, Vinylacetylen sowie $C_5$- und $C_6$-Acetylene mit oder ohne zusätzliche olefinische Bindung. In besonderer Weise sei die selektive Hydrierung von Vinylacetylen zu Butadien-1,3 erwähnt.

Die genannten acetylenischen Verbindungen liegen vielfach im Gemisch mit olefinischen, diolefinischen und/oder gesättigten Kohlenwasserstoffen vor, die hauptsächlich 3 – 6 C-Atome enthalten. Vielfach liegen die acetylenischen Verbindungen in noch engeren Fraktionierungsschnitten vor, wie sie üblicherweise in petrochemischen Anlagen der Raffinerien im Anschluss an Krackprozesse gewonnen werden. Die Erfindung betrifft daher weiterhin die Verwendung der erfindungsgemässen Katalysatoren für die selektive Hydrierung von acetylenischen zu olefinischen Verbindungen.

Eine solche Hydrierung kann in der Flüssig-, Riesel-, Gas- oder Mischphase erfolgen, bevorzugt in der Riesel- und in der kombinierten Flüssig-Mischphase. Die Reaktionstemperatur liegt beispielsweise bei 273 – 373 K, bevorzugt 303 – 343 K. Der Reaktionsdruck beträgt beispielsweise 1 – 20 bar, bevorzugt 2 – 9 bar, besonders bevorzugt 3 – 8 bar.

Die Katalysatorbelastung wird beispielsweise auf eine LHSV (liquid hourly space velocity) von 5 – 100, bevorzugt 10 – 50, besonders bevorzugt 15 – 25 m$^3$ Substrat/m$^3$ Katalysator · Stunde eingestellt. Die H$_2$-Menge wird entsprechend den zu hydrierenden Stoffmengen und dem Grad der acetylenischen Ungesättigtheit zudosiert und kann beispielsweise bei 0,5 – 5 Mol H$_2$/Mol acetylenisches Substrat, bevorzugt bei 0,7 – 2 Mol/Mol, besonders bevorzugt 0,8 – 1,5 Mol/Mol liegen.

Der Hydrierreaktor wird zur Abführung der Reaktionswärme bei einem Röhrenreaktor oder Doppelmantelreaktor üblicherweise mit einem Wärmeaustauschermedium, beispielsweise mit Wasser, gekühlt. Zur Vermeidung von Temperaturspitzen sind besonders solche Reaktortypen geeignet, bei denen die Hydrierwärme durch das Einsatzprodukt selbst aufgenommen und abgeführt wird, beispielsweise Reaktoren, bei denen das Hydrierprodukt zum grössten Teil dem Einsatzprodukt wieder beigemischt wird. Die Menge des zurückgeführten Reaktorproduktes zum neu eingesetzten Strom mit acetylenischen Verbindungen beträgt beispielsweise 5:1 bis 20:1, bevorzugt 10:1 bis 15:1. Weiterhin besonders geeignet sind solche Reaktoren bei denen die Hydrierwärme durch Verdampfen eines Teils der eingesetzten Kohlenwasserstoffe abgeführt wird (Typ des Siedereaktors).

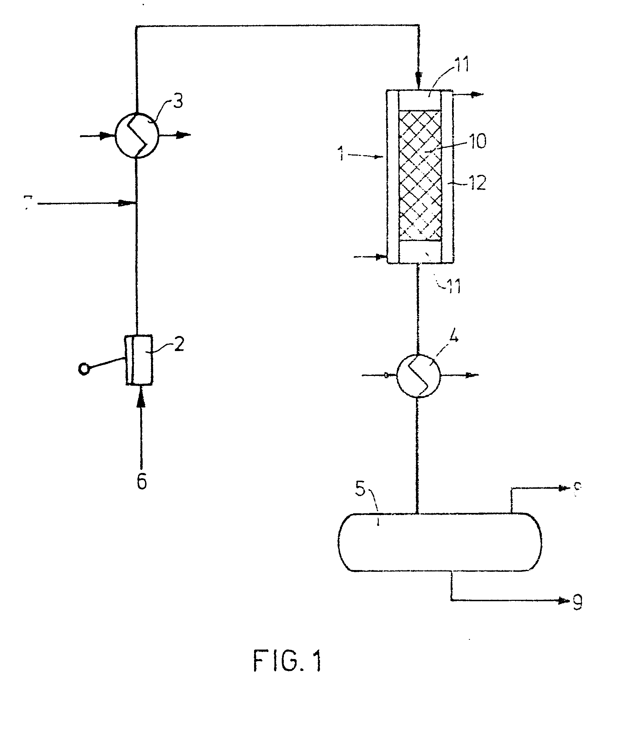

Die Hydrierung kann beispielsweise in einer aus der beigefügten Abbildung hervorgehenden Anordnung durchgeführt werden. Hierbei haben die Ziffern dieser Abbildung folgende Bedeutung: 1 Hydrierreaktor mit Doppelmantelkühlung, 2 Pumpe, 3 Vorwärmer, 4 Kühler, 5 Abscheider, 6 Einsatz-Kohlenwasserstoffstrom mit acetylenischen Verbindungen, 7 Wasserstoff, 8 Restgas, 9 Hydrierprodukt, 10 Hydrierkontakt, 11 obere und untere Schicht aus inertem Trägermaterial und 12 Eintritt und Austritt eines Kühlmediums.

Wie die folgenden Beispiele zeigen, liegt der Vorteil der erfindungsgemässen Katalysatoren in einer erheblich höheren Selektivität der Hydrierung von acetylenischen Verbindungen zu olefinischen Verbindungen, beispielsweise des Vinylacetylens zu Butadien-1,3, und damit in einer deutlich höheren Olefin-(z.B. Butadien-1,3-) ausbeute. Die mit Hilfe der erfindungsgemässen Katalysatoren erzielbaren Butadien-1,3- Ausbeuten aus C$_4$-Schnitten von Steam-Crackern liegen 20 – 50%, in manchen Fällen sogar mehr als 100% höher als die Ausbeuten mit Katalysatoren nach dem Stand der Technik. Als Kriterien für die Leistung der erfindungsgemässen Katalysatoren werden in den folgenden Beispielen dementsprechend herangezogen:

a) der Umsatz der acetylenischen Verbindungen in %;

b) die Selektivität des Umsatzes von acetylenischer zu olefinischer Verbindung und

c) die Zunahme der gewünschten olefinischen Verbindung (z.B. des Butadiens-1,3).

Beispiel 1
Katalysator:

Ein handelsüblicher Katalysator mit 3 g Pd/l auf $\alpha$-Al$_2$O$_3$-Tabletten, Grösse 3 mm × 3 mm, und einer spez. Oberfläche von ca. 3,5 m$^2$/g wurde

a) undotiert

b) wie folgt dotiert

zur selektiven Hydrierung eingesetzt:

70 ml Katalysator wurden zusammen mit 210 ml einer 0,7%igen wässrigen KNO$_3$-Lösung in einem Rotationskolbenverdampfer bei 353 K und 80 mbar bis zur Trockne erhitzt. Anschliessend wurde der Katalysator bei 80 mbar und 393 K 4 Stunden getrocknet und bei 773 K 5 Stunden kalziniert.

Hydrierversuche:

Je 60 ml des undotierten und des mit K$_2$O dotierten Katalysators wurden nacheinander in der in der Abbildung beschriebenen Hydrierapparatur eingesetzt.

Als Reaktor diente ein Doppelmantelrohr aus V$_2$A mit folgenden Abmessungen des Innenrohres:

| | |
|---|---|
| Länge | 350 mm |
| Innendurchmesser: | 15 mm |

Die Reaktionsbedingungen betrugen:
LHSV: 20 m$^3$/m$^3$ Katalysator.h
H$_2$-Menge: 0,7 Mol H$_2$/Mol Acetylene + Butadien-1,2
Reaktortmperatur: 313 K
Reaktordruck: 8 bar

Das Einsatzprodukt war ein C$_4$-Kohlenwasserstoffschnitt aus einer extraktiven C$_4$-Acetylenabtrennung mit folgender Zusammensetzung:

| | |
|---|---|
| Vinylacetylen | 5,0 Vol.-% |
| Ethylacetylen | 1,0 Vol.-% |
| Butadien-1,2 | 0,2 Vol.-% |
| Butadien-1,3 | 0,7 Vol.-% |
| Butene + Butane | Rest |

Nach einer Laufzeit der Hydrierung von 4 Stunden wurden Analysenproben zwecks Beurteilung des Hydrierergebnisses entnommen. Hierbei wurden folgende Ergebnisse erzielt:

| Verwendeter Katalysator: | | undotiert | dotiert mit ca. 1% K$_2$O |
|---|---|---|---|
| Umsatz | % | 44,9 | 52,4 |
| Selektivität | % | 39,3 | 59,5 |
| Butadien-1,3-Zunahme | % | 105,7 | 254,3 |

Beispiel 2
Katalysator:

Ein handelsüblicher Katalysator mit 3 g Pd/l auf $\alpha$-Al$_2$O$_3$-Kugeln, Grösse 3 – 5 mm, und einer spez. Oberfläche von ca. 5 m$^2$/g wurde
a) undotiert
b) wie folgt dotiert
zur selektiven Hydrierung eingesetzt:

70 ml Katalysator wurden zusammen mit 210 ml einer 0,92%igen wässrigen KNO$_3$-Lösung in einem Rotationskolbenverdampfer bei 353 K und 80 mbar bis zur Trockne erhitzt. Anschliessend wurde der Katalysator bei 80 mbar und 393 K 4 Stunden getrocknet und bei 773 K 5 Stunden kalziniert.

Hydrierversuche:

Je 60 ml des undotierten und des mit K$_2$O dotierten Katalysators wurden nacheinander in der in der Abbildung beschriebenn Hydrierapparatur eingesetzt.

Einsatzprodukt, Reaktionsbedingungen, Laufzeiten etc. entsprachen denen des Beispiels 1.

Es wurden folgende Ergebnisse erzielt:

| Verwendeter Katalysator: | | undotiert | dotiert mit ca. 1,3% K$_2$O |
|---|---|---|---|
| Umsatz | % | 22,2 | 52,0 |
| Selektivität | % | 65,6 | 80,4 |
| Butadien-1,3-Zunahme | % | 99,1 | 283,7 |

Beispiel 3
Katalysator:

Ein handelsüblicher Katalysator mit 5 g Pd/l auf $\alpha$-Al$_2$O$_3$-Extrudaten, Grösse 4 mm × 6 mm, und einer spez. Oberfläche von ca. 4 m$^2$/g wurde
a) undotiert
b) wie folgt dotiert
zur selektiven Hydrierung eingesetzt:

70 ml Katalysator wurden zusammen mit 200 ml einer 0,43%igen wässrigen KNO$_3$-Lösung in einem Rotationskolbenverdampfer bei 353 K und 80 mbar bis zur Trockne erhitzt. Anschliessend wurde der Katalysator bei 80 mbar und 393 K 4 Stunden getrocknet und 5 Stunden bei 773 K kalziniert.

Hydrierversuche:

Je 60 ml des undotierten und des mit K$_2$O dotierten Katalysators wurden nacheinander in der in der Abbildung beschriebenen Hydrierapparatur eingesetzt.

Einsatzprodukt, Reaktionsbedingungen, Laufzeiten etc. entsprachen denen des Beispiels 1.

Es wurden folgende Ergebnisse erzielt:

| Verwendeter Katalysator: | | undotiert | dotiert mit ca. 0,6% K$_2$O |
|---|---|---|---|
| Umsatz | % | 49,2 | 47,6 |
| Selektivität | % | 45,7 | 72,8 |
| Butadien-1,3-Zunahme | % | 149,6 | 269,2 |

## Patentansprüche

1. Hydrierkatalysatoren aus 0,1 – 60 Gew.-% eines hydrieraktiven Metalls oder einer hydrieraktiven Metallverbindung der VIII. Nebengruppe des Periodensystems der Elemente auf einem inerten Träger mit 0,1 – 10 Gew.-% K$_2$O sowie gegebenenfalls 0,001 – 10 Gew.-% eines Zusatzstoffes aus der Gruppe Calcium, Magnesium, Barium, Lithium, Natrium, Vanadium, Silber, Gold, Kupfer oder Zink, alles bezogen auf das Gesamtgewicht des Katalysators, wobei die K$_2$O-Dotierung auf eine Katalysator-Vorstufe aus der hydrieraktiven Komponente, dem Träger und gegebenenfalls dem Zusatzstoff aufgebracht wird.

2. Hydrierkatalysatoren nach Anspruch 1 mit 0,3 bis 3 Gew.-% K$_2$O.

3. Hydrierkatalysatoren nach Anspruch 1 mit 0,5 bis 2 Gew.-% K$_2$O.

4. Hydrierkatalysatoren nach Anspruch 1 bis 3 mit einem Edelmetall der VIII. Nebengruppe des Periodensystems der Elemente als hydrieraktiver Komponente.

5. Hydrierkatalysator nach Anspruch 1 bis 4 mit 0,1 bis 5 Gew.-% des Edelmetalls, bezogen auf das Gesamtgewicht des Katalysators.

6. Verfahren zur Herstellung eines Hydrierkatalysators nach Anspruch 1 und 5, dadurch gekennzeichnet, dass man 0,1 – 60 Gew.-% eines hydrieraktiven Metalls oder einer hydrieraktiven Metallverbindung und gegebenenfalls 0,001 – 10 Gew.-% eines Zusatzstoffes in an sich bekannter Weise auf einen inerten Träger bringt und anschliessend 0,1 – 10 Gew.-% einer wasserlöslichen Kaliumverbindung, gerechnet als K$_2$O, auf die beschriebene Katalysator-Vorstufe bringt, trocknet und durch Kalzinierung fixiert, wobei alle Gewichtsangaben auf das Gesamtgewicht der Hydrierkatalysatoren bezogen sind.

7. Verfahren nach Anspruch 6, dadurch gekennzeichnet, dass das Aufbringen der Kaliumverbindung durch Tränken erfolgt.

8. Verwendung des Hydrierkatalysators nach Anspruch 1 zur selektiven Hydrierung von acetylenischen zu olefinischen Verbindungen.

9. Verwendung des Hydrierkatalysators nach Anspruch 8, dadurch gekennzeichnet, dass die acetylenischen Verbindungen in einem Kohlenwasserstoffstrom enthalten sind, der ausserdem noch olefinische, diolefinische und/oder gesättigte Kohlenwasserstoffe, hauptsächlich im C$_3$–C$_6$-Bereich, enthält.

10. Verwendung des Hydierkatalysators nach Anspruch 8 zur selektiven Hydrierung von Vinylacetylen zu Butadien-1,3 in Gegenwart von olefinischen, diolefinischen und/oder gesättigten Kohlenwasserstoffen, hauptsächlich im C$_4$-Bereich.

## Revendications

1. Catalyseurs d'hydrogénation constitués de 0,1 à 60% en poids d'un métal doué d'activité hydrogénante ou d'un composé de métal doué d'activité hydrogénante du sous-groupe VIII du Système Périodique de Classification des Eléments sur un support inerte, avec 0,1 à 10% en

poids de $K_2O$ ainsi que, le cas échéant, 0,001 à 10% en poids d'un additif du groupe calcium, magnésium, baryum, lithium, sodium, vanadium, argent, or, cuivre ou zinc, le tout par rapport au poids total du catalyseur, le dopage au $K_2O$ étant appliqué sur un stade préalable du catalyseur formé du composant doué d'activité hydrogénante, du support et le cas échéant de l'additif.

2. Catalyseurs d'hydrogénation suivant la revendication 1, contenant 0,3 à 3% en poids de $K_2O$.

3. Catalyseurs d'hydrogénation suivant la revendication 1, contenant 0,5 à 2% en poids de $K_2O$.

4. Catalyseurs d'hydrogénation suivant la revendication 1 à 3, contenant un métal noble du sous-groupe VIII du Système de Classification Périodique des Eléments comme composant doué d'activité hydrogénante.

5. Catalyseurs d'hydrogénation suivant les revendications 1 à 4 contenant 0,1 à 5% en poids du métal noble, par rapport au poids total du catalyseur.

6. Procédé de production d'un catalyseur d'hydrogénation suivant les revendications 1 et 5, caractérisé en ce qu'on applique d'une manière connue sur un support inerte 0,1 à 60% en poids d'un métal doué d'activité hydrogénante ou d'un composé de métal doué d'activité hydrogénante et le cas échéant, 0,001 à 10% en poids d'un additif, puis on applique 0,1 à 10% en poids d'un composé de potassium soluble dans l'eau, exprimé en $K_2O$, sur le stade préalable décrit du catalyseur, on sèche et on fixe par calcination, toutes les indications de poids se rapportant au poids total des catalyseurs d'hydrogénation.

7. Procédé suivant le revendication 6, caractérisé en ce que l'application du composé de potassium est effectuée par imprégnation.

8. Utilisation du catalyseur d'hydrogénation suivant la revendication 1 pour l'hydrogénation sélective de composés acétyléniques en composés oléfiniques.

9. Utilisation du catalyseur d'hydrogénation suivant la revendication 8, caractérisée en ce que les composés acétyléniques sont contenus dans un courant d'hydrocarbures qui contient en outre principalement des hydrocarbures oléfiniques, dioléfiniques et/ou saturés, principalement dans l'intervalle de $C_3$ à $C_6$.

10. Utilisation du catalyseur d'hydrogénation suivant la revendication 8 pour l'hydrogénation sélective de vinylacétylène en butadiène-1,3 en présence d'hydrocarbures oléfiniques, dioléfiniques et/ou saturés, principalement de l'ordre de $C_4$.

**Claims**

1. Hydrogenation catalysts consisting of 0.1 – 60% by weight of a hydrogenating metal or of a hydrogenating metal compound of subgroup VIII of the periodic system of the elements on an inert support, containing 0.1 – 10% by weight of $K_2O$ and, optionally, 0.001 – 10% by weight of an additive from the group comprising calcium, magnesium, barium, lithium, sodium, vanadium, silver, gold, copper and zinc, in each case based on the total weight of the catalyst, the $K_2O$ doping being applied to a catalyst precursor consisting of the hydrogenating component, the support and, optionally, the additive.

2. Hydrogenation catalysts according to Claim 1 containing 0.3 to 3% by weight of $K_2O$.

3. Hydrogenation catalysts according to Claim 1 containing 0.5 to 2% by weight of $K_2O$.

4. Hydrogenation catalysts according to Claim 1 to 3 containing a noble metal of subgroup VIII of the periodic system of the elements as the hydrogenating component.

5. Hydrogenation catalyst according to Claim 1 to 4 containing 0.1 to 5% by weight of the noble metal, based on the total weight of the catalyst.

6. Process for the preparation of a hydrogenation catalyst according to Claim 1 and 5, characterised in that 0.1 –60% by weight of a hydrogenating metal or of a hydrogenting metal compound and, optionally, 0.001 – 10% by weight of an additive are applied, in a known manner, to an inert support and then 0.1 – 10% by weight of a water-soluble potassium compound, calculated as $K_2O$, are applied to the above-described catalyst precursor, dried and fixed by calcination, all the weight data being based on the total weight of the hydrogenation catalysts.

7. Process according to Claim 6, characterised in that the potassium compound is applied by impregnation.

8. Use of the hydrogenation catalyst according to Claim 1 for the selective hydrogenation of acetylenic compounds to give olefinic compounds.

9. Use of the hydrogenation catalyst according to Claim 8, characterised in that the acetylenic compounds are contained in a hydrocarbon stream which also contains olefinic, diolefinic and/or saturated hydrocarbons, mainly of the $C_3$–$C_6$ range.

10. Use of the hydrogenation catalyst according to Claim 8 for the selective hydrogenation of vinylacetylene to give 1,3-butadiene in the presence of olefinic, diolefinic and/or saturated hydrocarbons, mainly of the $C_4$ range.

FIG. 1